(19) Europäisches Patentamt / European Patent Office / Office européen des brevets

(11) **EP 2 588 855 B1**

(12) **FASCICULE DE BREVET EUROPEEN**

(45) Date de publication et mention de la délivrance du brevet:
**19.03.2014 Bulletin 2014/12**

(21) Numéro de dépôt: **11730927.8**

(22) Date de dépôt: **23.06.2011**

(51) Int Cl.:
**G01N 33/12** *(2006.01)*

(86) Numéro de dépôt international:
**PCT/EP2011/060556**

(87) Numéro de publication internationale:
**WO 2012/130340 (04.10.2012 Gazette 2012/40)**

(54) **PROCÉDÉ D'ÉVALUATION DE LA QUANTITÉ DE MÉTHANE PRODUITE PAR UN RUMINANT DIT "À VIANDE"**

VERFAHREN ZUR BEURTEILUNG DER VON EINEM WIEDERKÄUER FÜR DIE FLEISCHPRODUKTION PRODUZIERTEN MENGE AN METHAN

METHOD FOR EVALUATING THE QUANTITY OF METHANE PRODUCED BY A RUMINANT USED FOR MEAT PRODUCTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.06.2010 FR 1055243**

(43) Date de publication de la demande:
**08.05.2013 Bulletin 2013/19**

(73) Titulaire: **Valorex**
**35210 Combourtille (FR)**

(72) Inventeurs:
• **WEILL, Pierre**
**F-35770 Vern Sur Seiche (FR)**
• **CHESNEAU, Guillaume**
**F-35133 Luitre (FR)**
• **GUERIN, Aude**
**F-35140 Saint Hilaire Des Landes (FR)**

(74) Mandataire: **Regimbeau**
**Espace Performance Bâtiment K**
**35769 St-Grégoire Cedex (FR)**

(56) Documents cités:
**FR-A1- 2 933 191**

• **J. L. ELLIS; E. KEBREAB; N. E. ODONGO; K. BEAUCHEMIN; S. MCGINN; J. D. NKRUMAH; S. S. MOORE; R. CHRISTOPHERSON; ET AL.: "Modeling methane production from beef cattle using linear and nonlinear approaches", JOURNAL OF ANIMAL SCIENCE, vol. 87, no. 4, 19 décembre 2008 (2008-12-19), pages 1334-1345, XP002619186, DOI: 10.2527/jas.2007-0725**
• **GRAINGER C ET AL: "Methane Emissions from Dairy Cows Measured Using the Sulfur Hexafluoride (SF6) Tracer and Chamber Techniques", JOURNAL OF DAIRY SCIENCE, AMERICAN DAIRY SCIENCE ASSOCIATION, US, vol. 90, no. 6, 1 juin 2007 (2007-06-01), pages 2755-2766, XP026955944, ISSN: 0022-0302 [extrait le 2007-06-01]**
• **J. A. N. MILLS; E. KEBREAB; C. M. YATES; L. A. CROMPTON; S. B. CAMMELL; M. S. DHANOA; R. E. AGNEW; J. FRANCE: "Alternative approaches to predicting methane emissions from dairy cows", JOURNAL OF ANIMAL SCIENCE, vol. 81, no. 12, décembre 2003 (2003-12), pages 3141-3150, XP002619185,**

**Description**

La présente invention est relative à un procédé d'évaluation de la quantité de méthane liée à l'élevage des ruminants élevés pour leur viande utilisant la composition en acides gras des lipides des viandes.

Le méthane est un gaz à effet de serre qui participe au réchauffement climatique.

Son pouvoir réchauffant est 21 fois celui du gaz carbonique. Ainsi, selon les experts, le méthane participe pour 20% à l'ensemble des gaz à effet de serre conduisant au réchauffement climatique.

La moitié de ce méthane, provient de l'agriculture (soit 10% des gaz à effet de serre au niveau mondial).

Et, la majorité du méthane d'origine agricole (70% en Europe) est du méthane entérique émis par les animaux d'élevage lors de leur digestion.

Les ruminants contribuent en France pour 98% à cet apport de méthane entérique.

En effet, les processus fermentaires chez les ruminants conduisent à de fortes émissions de méthane.

Environ la moitié de ce méthane entérique des ruminants provient des femelles laitières et l'autre moitié des troupeaux élevés spécifiquement pour la production de viande.

Ainsi de nombreux experts appellent soit à diminuer la consommation de viandes issues de ruminants (principalement de boeuf) alors que d'autres experts appellent à choisir des modes de production moins émetteurs de méthane.

Pour conforter des modes de production « moins émetteurs de méthane », il est nécessaire de disposer de mesures de méthane liées au mode de production.

Selon les types de production, la quantité de méthane émise par kilo de viande produite pourrait varier de 1 à 6.

Les méthodes existantes d'évaluation de cette quantité de méthane produite par kilo de viande sont difficiles à mettre en oeuvre.

Elles sont en effet basées sur des mesures en fermes expérimentales (méthodes de chambre calorimétrique, ou mesure indirecte dite « à l'hexafluorure de soufre ») qui s'avèrent impossibles à mettre en place de façon systématique.

D'autres méthodes utilisent des équations de "prédiction" qui comportent, pour être précises, de nombreuses données individuelles spécifiques aux animaux producteurs de viande, telles que leur âge, leur vitesse de croissance, leur poids, la quantité de ration ingérée au cours de la vie et la composition de cette ration à chaque stade de leur vie.

Dans tous les cas, à ce jour, il est impossible de connaître la quantité de méthane émise lors de la production d'un kilogramme de viande, si l'on ne dispose pas de données précises sur l'animal dont est issue cette viande.

Le présent demandeur a déposé un brevet français (FR 2 933 191) relatif à un procédé d'évaluation du méthane produit par litre de lait de vaches laitières qui fait usage d'une méthode rapide qui lie la composition en acides gras du lait à la production de méthane.

Dans ce contexte, la problématique est relativement simple pour des vaches laitières puisque le lien entre la quantité émise quotidiennement par la vache et la quantité d'acides gras présents quotidiennement dans le lait ont un lien direct.

Par ailleurs, les prélèvements sur le lait s'effectuent sur un animal vivant dont les performances sont connues.

A ce jour, il existe donc encore un besoin non satisfait d'un procédé permettant d'évaluer l'"empreinte méthane" d'une viande et, par voie de conséquence, d'orienter les producteurs vers des méthodes respectueuses des contraintes liées au réchauffement climatique.

La présente invention vise à répondre à cette attente.

Ainsi, l'objet de l'invention concerne un procédé d'évaluation de la quantité de méthane produite par un ruminant dit "à viande", tel qu'un bovin, c'est-à-dire d'un animal élevé puis abattu pour la commercialisation de sa viande, caractérisé en ce qu'il consiste à déterminer la quantité d'au moins un acide gras AG contenu dans un tissu de référence, à savoir un muscle ou un tissu adipeux, prélevé sur ledit ruminant mort (en g d'AG/Kg de tissu) et à calculer ladite quantité de méthane (en g de $CH_4$/Kg de viande de l'animal) selon une équation qui est fonction de ladite quantité dudit AG, de la catégorie dudit animal, de son âge et de son poids, ces trois derniers critères étant déterminés au moment de son abattage, cette equation s'écrivant :

$$CH4 \text{ (g/Kg de viande de l'animal)} = [[[(\text{Age en mois}) * \text{Coef 1}] + \text{Coef 2}] * 1000 * \text{Taux de lipides (en \%)} * \text{Teneur en AG (en \% des lipides totaux)}] * 1000 / \text{Poids de l'animal (en kg de viande)},$$

équation dans laquelle :

- les coefficients Coef 1 et Coef 2 sont des nombres dont la valeur est fonction de la nature du tissu de référence et de la catégorie de l'animal.

Ainsi, grâce à ce procédé, on peut mesurer cette « empreinte méthane » d'une viande de façon rapide, peu coûteuse, systématique, en disposant de quelques éléments seulement, à savoir :

- l'âge de l'animal dont provient le tissu et sa catégorie (boeuf, génisse, vache, etc...),
- un échantillon de tissu de cet animal (échantillon de muscle dit "long dorsal" ("longissimus dorsi"), par exemple).

L'âge de l'animal figure toujours parmi les données de traçabilité qui accompagnent les carcasses de viande.
L'échantillon de tissu est destiné à une mesure du contenu en au moins un acide gras des lipides de cet échantillon.
On notera que de nombreuses données bibliographiques lient les mécanismes de la méthanogenèse ruminale à ceux de la lipogenèse dans le foie et le tissu adipeux.
De plus, des données expérimentales sont disponibles, qui lient le mode de production des animaux élevés pour leur viande (type d'élevage et composition de la ration) à la composition lipidique des viandes, notamment des données relatives au :

i. Pourcentage de lipides dans le morceau considéré par extraction éthérée ;
ii. Profil en acides gras de ces lipides mesuré par chromatographie en phase gazeuse ou par d'autres méthodes.

Enfin, une base de données permet de prédire la teneur des acides gras mineurs à partir de certains acides gras majeurs et de groupes d'acides gras dans des échantillons de viandes ou de tissus adipeux issus de ruminants.
Selon d'autres caractéristiques avantageuses et non limitatives de ce procédé :
- ledit tissu de référence est le muscle "longissimus dorsi" (long dorsal) ;
- ladite détermination de la quantité d'AG est réalisée par analyse directe du tissu de référence ;
- ladite détermination de la quantité d'AG est réalisée par analyse d'un autre tissu, puis déduction de la quantité d'AG dudit muscle de référence par une équation de prédiction ;
- ledit AG est l'acide palmitique C16:0 ;
- ledit autre tissu est choisi parmi la bavette, la hampe et le rond de gite et la quantité d'acide palmitique dans le "longissimus dorsi" ($C16{:}0_{LD}$) est donné par l'une ou l'autre des équations suivantes :

$$C16{:}0_{LD} = 0.884*C16{:}0_{Bavette} + 2.240 \quad (r^2 = 0.855, n = 48, p < 0.001)$$

$$C16{:}0_{LD} = 1.053*C16{:}0_{Hampe} + 1.076 \quad (r^2 = 0.78, n = 67, p < 0.001)$$

$$C16{:}0_{LD} = 0.948*C16{:}0_{Rond\ de\ Gite} + 2.095 \quad (r^2 = 0.70, n = 25, p < 0.001)$$

équations dans lesquelles $C16{:}0_{Bavette}$, $C16{:}0_{Hampe}$ et $C16{:}0_{Rond}$ de gite sont les teneurs en acide palmitique des tissus correspondants, r est le coefficient de corrélation, n est le nombre d'échantillons testés et p l'indice de significativité.
- ledit AG est différent de l'acide palmitique, mais est fortement corrélé à celui-ci, avec un seuil de significativité p inférieur à 0,01 ;
- lesdits Coefficients 1 et 2 ont les valeurs suivantes :

|  | Coef 1 | Coef 2 |
|---|---|---|
| Jeune bovin | 1.511 +/- 0.506 | -13.782 +/- 5.0615) |
| Génisse | 0.555 +/- 0.1905 | -4.807 +/-1.907 |
| Boeuf | 0.885 +/- 0.278 | -7.522 +/- 2.779 |
| Vache allaitante | 0.582 +/- 0.235 | 0 |

- lesdits coefficients ont les valeurs suivantes :

|  | Coef 1 | Coef 2 |
|---|---|---|
| Jeune bovin | 1.507 | -13.792 |

(suite)

|  | Coef 1 | Coef 2 |
|---|---|---|
| Génisse | 0.556 | -5.108 |
| Boeuf | 0.8848 | -7.552 |
| Vache allaitante | 0.582 | 0.000 |

Dans l'ensemble de la présente demande, les termes suivants sont définis comme expliqué ci-dessous.

Génisse : femelle n'ayant pas eu de veau ;
Vache allaitante : femelle ayant eu au moins un veau et dont le lait a servi à le nourrir ;
Jeune bovin : mâle non castré de moins de 2 ans ;
Boeuf : mâle castré de plus de 2 ans.

D'autres caractéristiques et avantages de l'invention apparaîtront à la lecture de la description détaillée qui va suivre de certains modes de réalisation.
La problématique particulière des ruminants, et en particulier des bovins, élevés pour leur viande est la suivante.
Lors des phénomènes de rumination, les hydrates de carbone présents dans les rations des ruminants sont fermentés par les populations de microbes présentes dans le rumen.
Les polyosides végétaux des rations sont dégradés en oses qui sont alors fermentés avec production d'acides organiques (acides gras volatils ou AGV), d'hydrogène et de gaz carbonique.
Deux voies principales co-existent dans le rumen :

i. La voie qui aboutit à la formation d'acide acétique (abrégé C2 pour 2 atomes de carbone) et d'acide butyrique (abrégé C4 pour 4 atomes de carbone).
Cette voie de fermentation produit de l'hydrogène.
Et l'hydrogène produit est ensuite évacué en grande partie sous forme de méthane ($CH_4$) lors des éructations des ruminants.
ii. La voie qui aboutit à la formation d'acide propionique (abrégé C3 pour 3 atomes de carbone).

Cette voie, au contraire, consomme de l'hydrogène présent dans le rumen.
Ainsi, la production de méthane est un phénomène physiologique, lié aux processus fermentaires microbiens dans le rumen des animaux polygastriques.
On sait depuis longtemps que l'équilibre de ces deux voies est très variable en fonction de l'alimentation des animaux. Ainsi la composition de la ration des animaux oriente vers des voies de fermentation différentes :

- Consommatrices d'hydrogène donc réductrices de la production de méthane pour la voie C3 ;
  ou
- Productrices d'hydrogène, donc responsables de la production de méthane pour les voies de C2 et C4.

De nombreux facteurs alimentaires sont à prendre en compte pour prédire une orientation vers les voies C2 et C4 ou vers la voie C3. On peut citer la fibrosité de la ration, la quantité de concentrés, la teneur en cellulose, la teneur en amidon, la teneur en lipides, etc...
Parmi les lipides présents dans la ration des ruminants, un acide gras poly-insaturé de la famille n-3 ou Oméga 3, l'acide alpha-linolénique (nomenclature C18 :3 n-3) occupe une place à part pour plusieurs raisons :

- Il est le principal acide gras des fourrages pâturés (jusque 70% des acides gras -AG- pour de l'herbe de printemps).
- Il comprend 3 insaturations qui sont en grande partie hydrogénées dans le rumen, consommant ainsi une petite fraction de l'hydrogène produit par la voie de C2 et C4 et produisant des composés intermédiaires de la bio-hydrogénation et de l'acide stéarique (C18 :0).
- Son apport dans les régimes oriente les fermentations vers plus de C3 et moins de C2 et de C4.
- Depuis plus de 30 ans, une bibliographie abondante indique que son ajout dans les régimes réduit la quantité de méthane produit par les ruminants dans des conditions expérimentales (chambre calorimétrique).
- Le mécanisme de cette réduction de méthane passerait par un effet toxique de cet acide gras, (et/ou de certains de ses dérivés issus de la bio-hydrogénation dans le rumen) sur certains microbes du rumen impliqués dans production d'hydrogène, et donc dans les premières étapes de la méthanogenèse.

- Enfin, l'acide alpha-liolénique Oméga 3 est un acide gras dit « essentiel et indispensable » pour les animaux, car sa synthèse utilise des enzymes (Delta 12 et Delta 15 désaturases) qui ne sont présentes que dans le règne végétal. Si cet AG se retrouve dans la viande, il provient forcément de l'alimentation.

Ainsi, si cet acide gras (ou ses dérivés) se retrouve dans la viande, il pourrait être considéré comme le marqueur de pratiques alimentaires qui défavorisent la production de méthane. Car ces pratiques alimentaires favorisent la voie du C3 consommatrice d'hydrogène au détriment du C2 et du C4, voies productrices d'hydrogène et donc de CH4.

La lipogenèse est la synthèse de lipides (et particulièrement d'acides gras) à partir de précurseurs qui sont essentiellement des glucides chez les monogastriques et essentiellement l'Acide Gras Volatil (AGV) Acétique (C2) chez les ruminants.

Ainsi, l'élément indispensable à la synthèse des lipides de ruminants élevés pour la viande est l'acide acétique (C2) dont la production ruminale s'accompagne d'émission de CH4.

Les acides gras présents dans les tissus des ruminants peuvent avoir deux origines, à savoir :

- Endogènes, lorsqu'ils sont issus de la lipogenèse à partir du précurseur acétique (C2). Ce sont des acides gras saturés ou mono-insaturés.
- Exogènes, lorsqu'ils proviennent de l'alimentation et sont incorporés aux triglycérides ou aux autres fractions lipidiques (Phospholipides).

Parmi ceux-ci, on comptera notamment les acides gras poly-insaturés exclusivement exogènes (puisqu'aucun animal ne possède les enzymes nécessaires à leur synthèse).

Ainsi, l'acide linolénique C18 :3 n-3 et l'acide acétique -AGV en C2- sont au carrefour des mécanismes de la méthanogenèse et de la lipogenèse.

L'acide alpha - linolénique, parce que sa présence (ou celle de ses dérivés Oméga 3) dans les lipides du ruminant est l'indice de sa présence dans la ration, et donc d'une réduction de la production d'acide acétique et de méthane.

L'acide acétique, parce que sa présence est nécessaire à la synthèse de la majorité des acides gras saturés d'une part, et parce que sa production s'accompagne toujours de production de méthane.

L'acide palmitique est très majoritairement issu de la synthèse endogène à partir du précurseur C2. La quantité d'acide palmitique endogène est très directement liée à une disponibilité en précurseur acide acétique et donc à une production ruminale d'hydrogène puis de méthane.

La réduction de la teneur en acide palmitique dans les viandes est donc toujours liée à une réduction des émissions de méthane, si l'acide alpha-linolénique (principal acide gras des rations des ruminants) est l'acide gras dominant des rations

Les lipides des viandes des ruminants sont :

- soit des lipides de structure, constitutifs des membranes des cellules du muscle et des autres organes ;
- soit des lipides de réserve, présents dans des triglycérides dans un tissu adipeux interne (persillé des viandes) ou externe aux muscles (ou autres organes).

Le tissu adipeux est une « réserve d'énergie » pour l'animal, les acides gras (AG) du tissu adipeux sont donc régulièrement mobilisés lors du vieillissement de l'animal. D'autres AG issus de l'alimentation (endogènes via le C2) ou exogènes viennent à leur tour s'incorporer dans le tissu adipeux.

La quantité de lipides présente dans un muscle donné reflète dans tous les cas l'intensité des mécanismes de lipogenèse endogène (donc de production de C2 et par voie de conséquence de CH4) mais aussi de l'âge et de l'historique de l'animal, c'est-à-dire de l'intensité de ses mécanismes de renouvellement du tissu adipeux.

La quantité et la nature des acides gras présents dans la viande d'un animal d'un âge connu est elle-même fonction de la nature de l'alimentation de cet animal. Elle est donc le reflet de la production de méthane de cet animal au cours de sa vie.

EXEMPLES DE MISE EN OEUVRE DU PROCEDE

A l'abattage de l'animal, un prélèvement de viande est effectué de façon standardisée, par exemple, un prélèvement de muscle au niveau de la 6$^{ème}$ côte.

L'échantillon prélevé est alors analysé pour connaître :

- son taux de lipides ;
- le profil en acides gras de ses lipides totaux.

Cette analyse est effectuée par extraction éthérée pour les lipides totaux et par chromatographie en phase gazeuse pour les acides gras.

Par ailleurs, les données de traçabilité classique donneront l'âge de l'animal, son sexe et sa race.

Ainsi, nous disposons des données suivantes (exemples) :

Animal 1 : Muscle Long Dorsal

Type d'animal : jeune bovin de race limousine (race à viande).
Age : 17 mois.
Poids : 400 kg de viandes (= poids de carcasse en kg * rendement en viande en %).
Taux de lipides de l'échantillon : 2.5% (en poids).
Teneur en C 16 :0 : 25.0% (des AG totaux).

Alors, la quantité de méthane émise est calculée comme suit :

$$CH4 \text{ (g/kg de viande)} = [[[(\text{Age en mois})* \text{Coef 1}] + \text{Coef 2}] * 1000 * \text{Taux de lipides (en \%)} * \text{Teneur en C16:0 (en \% des AG totaux)}] *1000 / \text{Poids (en kg de viandes)}.$$

Pour les jeunes bovins de race à viande, les coefficients Coef 1 et Coef 2 ont les valeurs suivantes : Coef 1 = 1.511 et Coef 2 = -13.782

Ce qui donne une valeur : CH4 (g/kg de viande) = 185 g

Animal 1 : Muscle de la Hampe

Type d'animal : jeune bovin de race limousine (race à viande).
Age : 17 mois.
Poids : 400 kg de viandes (= poids de carcasse en kg * rendement en viande en %).
Taux de lipides de l'échantillon : 7.5% (en poids).
Teneur en C16 :0 : 24.5% (des AG totaux).
Quantité de méthane émise :

$$CH4 \text{ (g/kg de viande)} = [[[(\text{Age en mois})* \text{Coef 1}] + \text{Coef 2}] * 1000 * \text{Taux de lipides (en \%)} * \text{Teneur en C16:0 (en \% des AG totaux)}] *1000 / \text{Poids (en kg de viandes)}.$$

Pour les jeunes bovins de race à viande : Coef 1 = 0.514 et Coef2 = -4.70.

CH4 (g/kg de viande) = 185 g

Animal 2 : Muscle Long Dorsal

Type d'animal : jeune bovin de race limousine (race à viande).
Age : 17 mois.
Poids : 400 kg de viandes (= poids de carcasse en kg * rendement en viande en %).
Taux de lipides de l'échantillon : 2.5% (en poids).
Teneur en C16:0 : 23.0% (des AG totaux).
Quantité de méthane émise :

$$CH4 \text{ (g/kg de viande)} = [[[(\text{Age en mois})* \text{Coef 1}] + \text{Coef 2}] * 1000$$
$$* \text{ Taux de lipides (en \%)} * \text{Teneur en C16 :0 (en \% des AG totaux)}] *1000 / \text{Poids}$$
$$\text{(en kg de viandes)}.$$

Pour les jeunes bovins de race à viande: Coef 1 = 1.511 et Coef 2 = -13.782.
CH4 (g/kg de viande) = 170 g.

Animal 2 : Muscle de la Hampe

Type d'animal : jeune bovin de race limousine (race à viande).
Age : 17 mois.
Poids : 400 kg de viandes (= poids de carcasse en kg * rendement en viande en %).
Taux de lipides de l'échantillon : 7.5% (en poids).
Teneur en C16:0 : 22.5% (des AG totaux).
Quantité de méthane émise :

$$CH4 \text{ (g/kg de viande)} = [[[(\text{Age en mois})* \text{Coef 1}] + \text{Coef 2}] * 1000$$
$$* \text{ Taux de lipides (en \%)} * \text{Teneur en C16 :0 (en \% des AG totaux)}] *1000 / \text{Poids}$$
$$\text{(en kg de viandes)}.$$

Pour les jeunes bovins de race à viande : Coef 1= 0.514 et Coef 2 = - 4.70.
CH4 (g/kg de viande) = 170 g.
A partir d'essais et de données de la bibliographie, lesdits coefficients ont été calculés pour chaque catégorie d'animal et chaque muscle.
De façon préférentielle, les coefficients pour le muscle Long Dorsal ont les valeurs suivantes :

|  | Coef 1 | Coef 2 |
|---|---|---|
| Jeune bovin | 1.511 | -13.782 |
| Génisse | 0.555 | -4.807 |
| Boeuf | 0.885 | -7.522 |
| Vache allaitante | 0.582 | 0.000 |

Pour le muscle de la Hampe, ils ont préférentiellement les valeurs suivantes :

|  | Coef 1 | Coef 2 |
|---|---|---|
| Jeune bovin | 0.5142 | -4.700 |
| Génisse | 0.3356 | -2.9042 |
| Boeuf | 0.3011 | -2.5596 |
| Vache allaitante | 0.2671 | 0.000 |

A titre d'exemple, on donne ci-après les équations de prédiction utilisées quand le muscle testé n'est pas le long dorsal et que l'acide gras est l'acide palmitique. Cet autre tissu peut être choisi parmi la bavette, la hampe et le rond de gite et la quantité d'acide palmitique dans le "longissimus dorsi" ($C16:0_{LD}$) est donnée par l'une ou l'autre des équations suivantes :

$$C16:0_{LD} = 0.884 * C16:0_{Bavette} + 2.240 \quad (r^2 = 0.855, n = 48, p < 0.001)$$

$$C16:0_{LD} = 1.053 * C16:0_{Hampe} + 1.076 \quad (r^2 = 0.78, n = 67, p < 0.001)$$

$$C16:0_{LD} = 0.948 * C16:0_{Rond\ de\ Gite} + 2.095 \quad (r^2 = 0.70, n = 25, p < 0.001)$$

équations dans lesquelles $C16:0_{Bavette}$, $C16:0_{Hampe}$ et $C16:0_{Rond}$ de gite sont les teneurs en acide palmitique des tissus correspondants, r est le coefficient de corrélation, n est le nombre d'échantillons testés et p le seuil de significativité.

De plus, on trouvera ci-après une matrice de corrélation (encore appelée de prédiction) permettant de calculer, par une équation de type Y=aX+b, avec Y=C16:0 (muscle i) et X=quantité d'un autre acide gras (même muscle i), la quantité d'acide palmitique de ce muscle. Ici, le muscle i est le "long dorsal".

Tableau 1

| Muscle = Long Dorsal cru (mg/100g) | | | | | | | | |
|---|---|---|---|---|---|---|---|---|
| Y<br>X | Moyenne | E.T. | R | R² | P value | N= | a= | b= |
| C16:0 | 789.37 | 479.66 | 0.00 | 0.00 | 0.00 | 0 | 0.000 | 0.000 |
| AGS | 1554.95 | 837.47 | 0.98 | 0.97 | 0.00 | 208 | 0.563 | -86.562 |
| AGMI | 1334.78 | 807.28 | 0.98 | 0.96 | 0.00 | 208 | 0.584 | 10.441 |
| C18:1 | 1201.25 | 715.86 | 0.98 | 0.96 | 0.00 | 208 | 0.656 | 1.199 |
| AGPI | 189.73 | 63.53 | 0.54 | 0.29 | 0.00 | 208 | 4.050 | 21.054 |
| AGPI n-3 | 34.10 | 18.36 | 0.47 | 0.22 | 0.00 | 208 | 12.309 | 369.697 |
| AGPIn-3-LC | 13.88 | 8.89 | 0.34 | 0.11 | 0.00 | 208 | 18.152 | 537.509 |
| CLA | 9.40 | 7.85 | 0.72 | 0.52 | 0.00 | 208 | 44.247 | 373.670 |
| ALA | 20.22 | 11.44 | 0.49 | 0.24 | 0.00 | 208 | 20.754 | 369.742 |
| C14:0 | 77.52 | 51.75 | 0.98 | 0.96 | 0.00 | 208 | 9.058 | 87.178 |
| C15:0iso | 5.69 | 3.53 | 0.87 | 0.76 | 0.00 | 208 | 118.806 | 113.845 |
| C15:0 | 13.18 | 6.88 | 0.91 | 0.82 | 0.00 | 208 | 63.306 | -45.021 |
| C16:0iso | 7.29 | 3.94 | 0.76 | 0.57 | 0.00 | 208 | 92.233 | 117.169 |
| C16:1 | 90.04 | 68.57 | 0.95 | 0.90 | 0.00 | 208 | 6.637 | 191.789 |
| C17:0iso | 12.53 | 6.50 | 0.92 | 0.84 | 0.00 | 208 | 67.753 | -59.374 |
| C16:0 | 789.37 | 479.66 | 0.00 | 0.00 | 0.00 | 0 | 0.000 | 0.000 |
| C17:0 | 31.35 | 16.62 | 0.93 | 0.86 | 0.00 | 208 | 26.840 | -51.935 |
| C17:1 | 18.66 | 11.95 | 0.96 | 0.91 | 0.00 | 208 | 38.366 | 73.322 |
| C18:0iso | 5.08 | 2.88 | 0.76 | 0.58 | 0.00 | 208 | 126.523 | 147.296 |
| C18:0 | 565.84 | 273.92 | 0.88 | 0.77 | 0.00 | 208 | 1.539 | -81.489 |
| C18:2 | 29.13 | 17.45 | 0.66 | 0.44 | 0.00 | 208 | 18.273 | 257.130 |
| C18:3 | 3.32 | 1.80 | 0.92 | 0.84 | 0.00 | 137 | 255.259 | 45.215 |
| C17:0anteiso | 20.22 | 9.99 | 0.86 | 0.73 | 0.00 | 207 | 41.213 | -43.617 |
| C15:0anteiso | 7.43 | 3.81 | 0.79 | 0.62 | 0.00 | 207 | 99.460 | 52.245 |
| C14:1 | 11.56 | 11.03 | 0.87 | 0.76 | 0.00 | 207 | 37.726 | 356.361 |
| C14:0iso | 2.96 | 1.71 | 0.92 | 0.85 | 0.00 | 102 | 245.730 | 38.147 |

(suite)

| Muscle = Long Dorsal cru (mg/100g) | | | | | | | | |
|---|---|---|---|---|---|---|---|---|
| X \ Y | Moyenne | E.T. | R | R² | P value | N= | a= | b= |
| C12:0 | 3.02 | 1.68 | 0.97 | 0.94 | 0.00 | 190 | 276.593 | -58.359 |
| C10:0 | 2.64 | 1.69 | 0.83 | 0.69 | 0.00 | 107 | 218.752 | 155.799 |

Par ailleurs, dans les tableaux 2 à 4 suivants sont données les matrices de corrélation permettant de calculer la quantité d'acide palmitique de ce muscle i, par le couplage d'une équation de type Y=aX+b, avec Y=C16:0 (muscle j) et X=quantité d'un autre acide gras (même muscle j), et une équation de prédiction du C16:0 du muscle i à partir du C16:0 du muscle j.

Ici, le muscle i est le long dorsal et le muscle j est respectivement la bavette, la hampe et le rond de gite.

Tableau 2

| Muscle = BAVETTE (mg/100g) | | | | | | | | |
|---|---|---|---|---|---|---|---|---|
| X \ Y | Moyenne | E.T. | R | R² | P value | N= | a= | b= |
| C16:0 | 603.75 | 350.03 | 0.00 | 0.00 | 0.00 | 0 | 0.000 | 0.000 |
| AGS | 1179.37 | 631.01 | 0.99 | 0.99 | 0.00 | 48 | 0.551 | -46.069 |
| AGMI | 1052.97 | 657.66 | 0.96 | 0.92 | 0.00 | 48 | 0.511 | 65.408 |
| C18:1 | 933.58 | 580.92 | 0.96 | 0.91 | 0.00 | 48 | 0.576 | 65.854 |
| AGPI | 184.95 | 81.65 | 0.67 | 0.45 | 0.00 | 48 | 2.887 | 69.750 |
| AGPI n-3 | 25.75 | 10.26 | 0.59 | 0.35 | 0.00 | 48 | 20.178 | 84.225 |
| AGPIn-6 | 127.50 | 59.68 | 0.57 | 0.33 | 0.00 | 48 | 3.359 | 175.542 |
| AGPIn-3-LC | 11.02 | 3.82 | 0.50 | 0.25 | 0.00 | 48 | 45.809 | 98.863 |
| CLA | 5.62 | 4.02 | 0.86 | 0.74 | 0.00 | 48 | 75.009 | 181.942 |
| ALA | 14.73 | 7.66 | 0.54 | 0.30 | 0.00 | 48 | 24.854 | 237.775 |
| LA | 108.15 | 51.69 | 0.59 | 0.34 | 0.00 | 48 | 3.972 | 174.199 |
| C14:0 | 67.67 | 44.32 | 0.98 | 0.97 | 0.00 | 48 | 7.776 | 77.544 |
| C15:0iso | 3.60 | 2.12 | 0.80 | 0.64 | 0.00 | 48 | 132.329 | 126.805 |
| C15:0 | 12.51 | 6.63 | 0.92 | 0.85 | 0.00 | 48 | 48.655 | -4.782 |
| C16:0iso | 6.85 | 3.57 | 0.85 | 0.73 | 0.00 | 48 | 83.802 | 29.347 |
| C16:1 | 76.11 | 50.31 | 0.98 | 0.96 | 0.00 | 48 | 6.802 | 86.044 |
| C17:0iso | 10.88 | 5.60 | 0.85 | 0.73 | 0.00 | 48 | 53.239 | 24.653 |
| C17:0 | 27.53 | 16.61 | 0.86 | 0.73 | 0.00 | 48 | 18.054 | 106.660 |
| C17:1 | 17.45 | 12.07 | 0.87 | 0.76 | 0.00 | 48 | 25.304 | 162.317 |
| C18:0iso | 5.16 | 3.13 | 0.87 | 0.76 | 0.00 | 48 | 97.778 | 99.249 |
| C18:0 | 394.37 | 190.99 | 0.94 | 0.89 | 0.00 | 48 | 1.729 | -78.148 |
| C18:2 | 24.16 | 15.70 | 0.66 | 0.44 | 0.00 | 48 | 14.809 | 246.016 |
| C18:2cj | 5.62 | 4.02 | 0.86 | 0.74 | 0.00 | 48 | 75.009 | 181.942 |
| C18:3 | 2.00 | 1.50 | 0.61 | 0.37 | 0.00 | 46 | 142.480 | 314.902 |
| C17:0anteiso | 22.08 | 14.68 | 0.80 | 0.64 | 0.00 | 48 | 19.121 | 1181.574 |
| C15:0anteiso | 6.86 | 3.77 | 0.83 | 0.69 | 0.00 | 48 | 76.782 | 76.753 |

(suite)

| Muscle = BAVETTE (mg/100g) | | | | | | | | |
|---|---|---|---|---|---|---|---|---|
| X \ Y | Moyenne | E.T. | R | R² | P value | N= | a= | b= |
| C14:1 | 11.93 | 10.06 | 0.93 | 0.87 | 0.00 | 48 | 32.377 | 217.450 |
| C14:0iso | 2.02 | 1.20 | 0.86 | 0.73 | 0.00 | 22 | 217.297 | 155.501 |
| C12:0 | 2.41 | 1.33 | 0.98 | 0.96 | 0.00 | 48 | 257.569 | -17.080 |
| C10:0 | 2.22 | 1.45 | 0.92 | 0.84 | 0.00 | 44 | 222.915 | 107.146 |

Tableau 3

| Muscle = HAMPE (mg/100g) | | | | | | | | |
|---|---|---|---|---|---|---|---|---|
| X \ Y | Moyenne | E.T. | R | R² | P value | N= | a= | b= |
| C16:0 | 1791.78 | 807.61 | 0.00 | 0.00 | 0.00 | 0 | 0.000 | 0.000 |
| AGS | 4310.14 | 1731.80 | 0.98 | 0.97 | 0.00 | 70 | 0.459 | -184.254 |
| AGMI | 3092.75 | 1456.98 | 0.94 | 0.89 | 0.00 | 70 | 0.522 | 177.956 |
| C18:1 | 2845.68 | 1341.17 | 0.94 | 0.88 | 0.00 | 70 | 0.566 | 180.761 |
| AGPI | 494.93 | 152.88 | 0.51 | 0.26 | 0.00 | 70 | 2.708 | 451.492 |
| AGPI n-3 | 69.11 | 27.72 | 0.46 | 0.21 | 0.00 | 70 | 13.305 | 872.315 |
| CLA | 20.61 | 15.06 | 0.77 | 0.60 | 0.00 | 70 | 41.411 | 938.128 |
| ALA | 49.37 | 23.52 | 0.48 | 0.23 | 0.00 | 70 | 16.486 | 977.860 |
| C14:0 | 190.48 | 88.86 | 0.97 | 0.93 | 0.00 | 70 | 8.781 | 119.263 |
| C15:0iso | 15.27 | 7.57 | 0.85 | 0.72 | 0.00 | 70 | 90.646 | 407.941 |
| C15:0 | 37.96 | 14.72 | 0.83 | 0.69 | 0.00 | 70 | 45.544 | 63.056 |
| C16:0iso | 24.06 | 10.65 | 0.82 | 0.68 | 0.00 | 70 | 62.359 | 291.505 |
| C16:1 | 150.14 | 72.08 | 0.94 | 0.89 | 0.00 | 70 | 10.583 | 202.871 |
| C17:0iso | 35.26 | 13.80 | 0.88 | 0.77 | 0.00 | 70 | 51.524 | -25.185 |
| C17:0 | 102.57 | 42.63 | 0.90 | 0.82 | 0.00 | 70 | 17.121 | 35.782 |
| C17:1 | 43.78 | 20.96 | 0.88 | 0.78 | 0.00 | 70 | 33.949 | 305.582 |
| C18:0iso | 16.64 | 7.45 | 0.90 | 0.81 | 0.00 | 70 | 97.735 | 165.180 |
| C18:0 | 1944.47 | 738.37 | 0.93 | 0.86 | 0.00 | 70 | 1.013 | -176.922 |
| C18:2 | 74.78 | 45.87 | 0.54 | 0.30 | 0.00 | 70 | 9.579 | 1075.396 |
| C18:2cj | 20.61 | 15.06 | 0.77 | 0.60 | 0.00 | 70 | 41.411 | 938.128 |
| C18:3 | 7.40 | 3.66 | 0.88 | 0.77 | 0.00 | 69 | 192.310 | 353.173 |
| C17:0anteiso | 67.74 | 26.74 | 0.76 | 0.58 | 0.00 | 70 | 22.964 | 236.193 |
| C15:0anteiso | 24.91 | 8.96 | 0.71 | 0.50 | 0.00 | 70 | 63.787 | 202.580 |
| C14:1 | 17.75 | 11.60 | 0.83 | 0.69 | 0.00 | 70 | 57.833 | 765.313 |
| C14:0iso | 7.91 | 3.56 | 0.78 | 0.61 | 0.00 | 49 | 176.833 | 508.393 |
| C12:0 | 7.58 | 3.13 | 0.98 | 0.96 | 0.00 | 64 | 249.034 | -167.120 |
| C10:0 | 7.03 | 3.87 | 0.90 | 0.80 | 0.00 | 57 | 186.333 | 420.298 |

Tableau 4

| Muscle = Rond de gite (mg/100g) | | | | | | | | |
|---|---|---|---|---|---|---|---|---|
| X ⟍ Y | Moyenne | E.T. | R | R² | P value | N= | a= | b= |
| C16:0 | 464.52 | 227.74 | 0.00 | 0.00 | 0.00 | 0 | 0.000 | 0.000 |
| AGS | 822.70 | 391.97 | 0.99 | 0.99 | 0.00 | 25 | 0.578 | -11.061 |
| AGMI | 944.87 | 448.35 | 0.98 | 0.96 | 0.00 | 25 | 0.498 | -6.176 |
| C18:1 | 827.98 | 392.43 | 0.98 | 0.96 | 0.00 | 25 | 0.569 | -6.427 |
| AGPI | 84.94 | 29.49 | 0.67 | 0.45 | 0.00 | 25 | 5.157 | 26.474 |
| CLA | 6.57 | 4.09 | 0.74 | 0.54 | 0.00 | 25 | 41.014 | 195.174 |
| ALA | 5.42 | 2.42 | 0.50 | 0.25 | 0.01 | 25 | 47.380 | 207.744 |
| LA | 35.74 | 10.74 | 0.70 | 0.49 | 0.00 | 25 | 14.872 | -66.985 |
| C14:0 | 40.88 | 22.87 | 0.96 | 0.93 | 0.00 | 25 | 9.608 | 71.761 |
| C15:0iso | 3.32 | 1.94 | 0.92 | 0.85 | 0.00 | 25 | 108.175 | 105.723 |
| C15:0 | 7.03 | 3.70 | 0.95 | 0.91 | 0.00 | 25 | 58.585 | 52.830 |
| C16:0iso | 4.49 | 2.47 | 0.93 | 0.86 | 0.00 | 25 | 85.472 | 80.750 |
| C16:1 | 80.23 | 40.82 | 0.92 | 0.84 | 0.00 | 25 | 5.126 | 53.252 |
| C17:0iso | 8.25 | 4.15 | 0.95 | 0.91 | 0.00 | 25 | 52.290 | 33.015 |
| C17:0 | 16.33 | 8.33 | 0.97 | 0.95 | 0.00 | 25 | 26.586 | 30.310 |
| C17:1 | 15.03 | 7.36 | 0.96 | 0.92 | 0.00 | 25 | 29.615 | 19.408 |
| C18:0iso | 3.70 | 1.71 | 0.99 | 0.98 | 0.00 | 25 | 132.169 | -24.326 |
| C18:0 | 244.80 | 113.38 | 0.95 | 0.90 | 0.00 | 25 | 1.906 | -2.145 |
| C18:2 | 17.01 | 9.08 | 0.81 | 0.65 | 0.00 | 25 | 20.195 | 120.991 |
| C18:2cj | 6.57 | 4.09 | 0.74 | 0.54 | 0.00 | 25 | 41.014 | 195.174 |
| C18:3 | 1.85 | 0.85 | 0.99 | 0.98 | 0.00 | 25 | 264.339 | -24.326 |
| C17:0anteiso | 12.14 | 6.17 | 0.98 | 0.95 | 0.00 | 25 | 36.005 | 27.577 |
| C15:0anteiso | 3.44 | 1.81 | 0.92 | 0.85 | 0.00 | 25 | 115.471 | 67.211 |
| C14:1 | 11.61 | 7.07 | 0.83 | 0.69 | 0.00 | 25 | 26.817 | 153.092 |
| C12:0 | 1.78 | 0.80 | 0.99 | 0.98 | 0.00 | 24 | 261.363 | -20.129 |

Dans l'ensemble de ces tableaux, les abréviations utilisées ont les significations suivantes :

E.T.    : écart type
R       : indice de corrélation
P       : seuil de significativité statistique
N       : nombre d'individus testés
AGS     : acides gras saturés
AGMI    : acides gras mono-insaturés
AGPI    : acides gras poly-insaturés
CLA     : acides linoléiques conjugués
ALA     : acide alpha-linolénique
LA      : acide linoléique

**Revendications**

1. Procédé d'évaluation de la quantité de méthane produite par un ruminant dit "à viande", tel qu'un bovin, c'est-à-dire d'un animal élevé puis abattu pour la commercialisation de sa viande, **caractérisé en ce qu'**il consiste à déterminer la quantité d'au moins un acide gras AG contenu dans un tissu de référence, à savoir un muscle ou un tissu adipeux, prélevé sur ledit ruminant mort (en g d'AG/Kg de tissu) et à calculer ladite quantité de méthane (en g de $CH_4$/Kg de viande de l'animal) selon une équation qui est fonction de ladite quantité dudit AG, de la catégorie dudit animal, de son âge et de son poids, ces trois derniers critères étant déterminés au moment de son abattage, cette équation s'écrivant :

$$CH4 \text{ (g/Kg de viande de l'animal)} = [[[(\text{Age en mois})* Coef\ 1\ ] + Coef\ 2\ ] * 1000 * \text{Taux de lipides (en \%)} * \text{Teneur en AG (en \% des lipides totaux)}] *1000 / \text{Poids de l'animal (en kg de viande)},$$

équation dans laquelle :

les coefficients Coef 1 et Coef 2 sont des nombres dont la valeur est fonction de la nature du tissu de référence et de la catégorie de l'animal.

2. Procédé d'évaluation selon la revendication 1, **caractérisé par le fait que** ledit tissu de référence est le muscle "longissimus dorsi" (long dorsal).

3. Procédé d'évaluation selon l'une des revendications 1 ou 2, **caractérisé par le fait que** ladite détermination de la quantité d'AG est réalisée par analyse directe du tissu de référence.

4. Procédé d'évaluation selon la revendication 2, **caractérisé par le fait que** ladite détermination de la quantité d'AG est réalisée par analyse d'un autre tissu, puis déduction de la quantité d'AG dudit muscle de référence par une équation de prédiction.

5. Procédé selon l'une des revendications précédentes, **caractérisé par le fait que** ledit AG est l'acide palmitique C16:0.

6. Procédé selon les revendications 4 et 5 prises en combinaison, **caractérisé par le fait que** ledit autre tissu est choisi parmi la bavette, la hampe et le rond de gite et que la quantité d'acide palmitique dans le "longissimus dorsi" ($C16:0_{LD}$) est donné par l'une ou l'autre des équations suivantes :

$$C16:0_{LD} = 0.884*C16:0_{Bavette} + 2.240 \quad (r^2=0.855, n=48, p<0.001)$$

$$C16:0_{LD} = 1.053*C16:0_{Hampe} + 1.076 \quad (r^2=0.78, n=67, p<0.001)$$

$$C16:0_{LD} = 0.948*C16:0_{Rond\ de\ Gite} + 2.095 \quad (r^2 = 0.70, n=25, p<0.001)$$

équations dans lesquelles $C16:0_{Bavette}$, $C16:0_{Hampe}$ et $C16:0_{Rond}$ de gite sont les teneurs en acide palmitique des tissus correspondants, r est le coefficient de corrélation, n est le nombre d'échantillons testés et p le seuil de significativité.

7. Procédé selon l'une des revendications 1 à 4, **caractérisé par le fait que** ledit AG est différent de l'acide palmitique, mais est fortement corrélé à celui-ci, avec un seuil de significativité p inférieur à 0,01.

8. Procédé selon les revendications 2 et 3 prises en combinaison, **caractérisé par le fait que** lesdits Coefficients 1 et 2 ont les valeurs suivantes :

|  | Coef 1 | Coef 2 |
|---|---|---|
| Jeune bovin | 1.511 +/- 0.506 | -13.782 +/- 5.0615) |
| Génisse | 0.555 +/- 0.1905 | -4.807 +/-1.907 |
| Boeuf | 0.885 +/- 0.278 | -7.522 +/- 2.779 |
| Vache allaitante | 0.582 +/- 0.235 | 0 |

**9.** Procédé selon la revendication 3, quand celle-ci est dépendante de la revendication 1 et que le tissu de référence est la hampe, **caractérisé par le fait que** lesdits coefficients ont les valeurs suivantes :

|  | Coef 1 | Coef 2 |
|---|---|---|
| Jeune bovin | 0.5142 | -4.7000 |
| Génisse | 0.3356 | -2.9042 |
| Boeuf | 0.3011 | -2.5596 |
| Vache allaitante | 0.2671 | 0.000 |

**Patentansprüche**

**1.** Verfahren zur Auswertung der Menge von Methan, die durch einen sogenannten 'zur Fleischproduktion gezüchteten' Wiederkäuer, wie ein Rind, d.h. ein Tier, das aufgezogen, dann für die Kommerzialisierung seines Fleisches geschlachtet wird, produziert wird, **dadurch gekennzeichnet, dass** es darin besteht, die Menge von mindestens einer Fettsäure AG, die in einem Referenzgewebe, nämlich einem Muskel oder einem Fettgewebe, das dem toten Wiederkäuer entnommen worden ist, enthalten ist, zu bestimmen (in g AG /kg Gewebe) und die Menge von Methan (in g $CH_4$/kg Fleisch des Tiers) gemäß einer Gleichung, die von der Menge der AG, der Kategorie dieses Tiers, von seinem Alter und seinem Gewicht abhängig ist, wobei diese letzteren drei Kriterien zum Zeitpunkt von dessen Schlachtung bestimmt werden, zu berechnen, wobei diese Gleichung lautet:

$CH_4$ (g/kg Fleisch des Tiers ) = [[[(Alter in Monaten) • Koeff. 1] + Koeff. 2] •

1000 • Anteil von Lipiden (in %) • Gehalt an AG (in % der gesamten Lipide)] •

1000 / Gewicht des Tiers (in kg Fleisch),

in welcher Gleichung:
die Koeffizienten Koeff. 1 und Koeff. 2 Zahlen sind, deren Wert von der Natur des Referenzgewebes und der Kategorie des Tiers abhängig ist.

**2.** Auswertungsverfahren nach Anspruch 1, **dadurch gekennzeichnet, dass** das Referenzgewebe der Muskel "longissimus dorsi" (long dorsal; langer Rückenstrecker) ist.

**3.** Auswertungsverfahren nach einem der Ansprüche 1 oder 2, **dadurch gekennzeichnet, dass** die Bestimmung der Menge von AG durch direkte Analyse des Referenzgewebes ausgeführt wird.

**4.** Auswertungsverfahren nach Anspruch 2, **dadurch gekennzeichnet, dass** die Bestimmung der Menge von AG durch Analyse eines anderen Gewebes, dann Ableitung der Menge von AG des Referenzmuskels durch eine Voraussagegleichung ausgeführt wird.

**5.** Verfahren nach einem der vorangegangenen Ansprüche, **dadurch gekennzeichnet, dass** die AG C16:0-Palmitinsäure ist.

**6.** Verfahren nach den Ansprüchen 4 und 5 zusammen genommen, **dadurch gekennzeichnet, dass** das andere Gewebe unter Flanke/Bauchlappen, Zwerchfell/Kronfleisch und Oberschale, Abschnitt 'Rond de Gite' nach französischer Zerlegung ausgewählt wird und dass die Menge von Palmitinsäure in dem 'Musculus longissimus dorsi' ($C16:0_{LD}$) angegeben wird durch die eine oder andere der folgenden Gleichungen:

$$C16:0_{LD} = 0{,}884 \cdot C16:0_{Flanke/Bauchlappen} + 2{,}240 \; (r^2 = 0{,}855, \, n = 48, \, p < 0{,}001)$$

$$C16:0_{LD} = 1{,}053 \cdot C16:0_{Zwerchfell/Kronfleisch} + 1{,}076 \; (r^2 = 0{,}78, \, n = 67, \, p < 0{,}001)$$

$$C16:0_{LD} = 0{,}948 \cdot C16:0_{Oberschale, \, 'Rond \, de \, Gite'} + 2{,}095 \; (r^2 = 0{,}70, \, n = 25, \, p < 0{,}001),$$

in welchen Gleichungen $C16:0_{Flanke/Bauchlappen}$, $C16:0_{Zwerchfell/Kronfleisch}$ und $C16:0_{oberschale, \, 'Rond \, de \, Gite'}$ die Gehalte an Palmitinsäure der entsprechenden Gewebe sind, r der Korrelationskoeffizient ist, n die Anzahl von getesteten Proben ist und p das Signifikanzniveau ist.

**7.** Verfahren nach einem der Ansprüche 1 bis 4, **dadurch gekennzeichnet, dass** die AG von Palmitinsäure verschieden ist, aber mit jener stark korreliert ist, mit einem Signifikanzniveau p unter 0,01.

**8.** Verfahren nach den Ansprüchen 2 und 3 zusammen genommen, **dadurch gekennzeichnet, dass** die Koeffizienten 1 und 2 die folgenden Werte haben:

|  | Koeff.1 | Koeff.2 |
|---|---|---|
| Junges Rind | 1,511 +/-0,506 | -13,782 +/- 5,0615) |
| Färse | 0,555 +/- 0,1905 | -4,807 +/- 1,907 |
| Rind | 0,885 +/- 0,278 | -7,522 +/- 2,779 |
| Kuh, welche ihr Kalb säugt | 0,582 +/- 0,235 | 0 |

**9.** Verfahren nach Anspruch 3, insoweit als jener von Anspruch 1 abhängig ist und dass das Referenzgewebe das Zwerchfell/Kronfleisch ist, **dadurch gekennzeichnet, dass** die Koeffizienten die folgenden Werte haben:

|  | Koeff. 1 | Koeff. 2 |
|---|---|---|
| Junges Rind | 0,5142 | -4,7000 |
| Färse | 0,3356 | -2,9042 |
| Rind | 0,3011 | -2,5596 |
| Kuh, welche ihr Kalb säugt | 0,2671 | 0,000 |

**Claims**

**1.** A method for determining the quantity of methane produced by a meat ruminant, such as a bovine, *i.e.,* an animal raised and then slaughtered for the sale of its meat, **characterized in that** it consists in measuring the quantity of at least one fatty acid (FA) contained in a reference tissue, namely muscle or adipose tissue, sampled from said ruminant after its death (in grams of fatty acids per kilogram of tissue) and calculating said quantity of methane (in grams of $CH_4$ per kilogram of meat from the animal) according to an equation that is a function of said quantity of said FA and the category, age and weight of said animal, wherein the latter three criteria are determined at the time of the slaughter of said animal, and wherein said equation is written as follows:

$$CH_4 \text{ (g/kg of meat of the animal)} = [[[(\text{Age in months})*\text{Coeff 1}] + \text{Coeff 2}]*1000*\text{Lipid content (in \%)}*\text{FA content (in \% of total lipids)}]*1000]/\text{Weight of the animal (in kg of meat)},$$

an equation in which:

the coefficients Coeff 1 and Coeff 2 are numbers whose value is a function of the nature of the reference tissue and the category of the animal.

2. The determining method of claim 1, **characterized in that** said reference tissue is the *longissimus dorsi* muscle.

3. The determining method of either claim 1 or claim 2, **characterized in that** said determination of FA quantity is carried out by direct analysis of the reference tissue.

4. The determining method of claim 2, **characterized in that** said determination of FA quantity is carried out by analysis of another tissue, and then deduction of the FA quantity of said reference muscle by a prediction equation.

5. The method according to any one of the preceding claims, **characterized in that** said FA is palmitic acid C16:0.

6. The method according to claims 4 and 5 in combination, **characterized in that** said other tissue is selected from flank, skirt and silverside and that the quantity of palmitic acid in the *longissimus dorsi* ($C16:0_{LD}$) is given by one of the following equations:

$$C16:0_{LD} = 0.884 * C16:0_{Flank} + 2.240 \quad (r^2 = 0.855, \quad n = 48, \quad p < 0.001);$$

$$C16:0_{LD} = 1.053 * C16:0_{Skirt} + 1.076 \quad (r^2 = 0.78, \quad n = 67, \quad p < 0.001);$$

$$C16:0_{LD} = 0.948 * C16:0_{Silverside} + 2.095 \quad (r^2 = 0.70, \quad n = 25, \quad p < 0.001);$$

equations wherein $C16:0_{Flank}$, $C16:0_{Skirt}$ and $C16:0_{Silverside}$ are the palmitic acid contents of the corresponding tissues, r is the correlation coefficient, n is the number of samples tested and p is the significance level.

7. The method of any one of claims 1 to 4, **characterized in that** said FA is different from palmitic acid, but strongly correlated with palmitic acid, with a significance level (p) less than 0.01.

8. The method of claims 2 and 3 in combination, **characterized in that** said Coefficients 1 and 2 have the following values:

|  | Coeff 1 | Coeff 2 |
|---|---|---|
| Young bovine | $1.511 \pm 0.506$ | $-13.782 \pm 5.0615$ |
| Heifer | $0.555 \pm 0.1905$ | $-4.807 \pm 1.907$ |
| Steer | $0.885 \pm 0.278$ | $-7.522 \pm 2.779$ |
| Nursing cow | $0.582 \pm 0.235$ | 0 |

9. The method of claim 3, when claim 3 is dependent on claim 1 and the reference tissue is skirt, **characterized in that** said coefficients have the following values:

|  | Coeff 1 | Coeff 2 |
|---|---|---|
| Young bovine | 0.5142 | -4.700 |
| Heifer | 0.3356 | -2.9042 |
| Steer | 0.3011 | -2.5596 |
| Nursing cow | 0.2671 | 0.000 |

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2933191